# EUROPEAN PATENT SPECIFICATION

(11) **EP 2 562 241 B1**
(45) Date of publication and mention of the grant of the patent: **17.05.2017**
(21) Application number: 12176958.2
(22) Date of filing: 18.07.2012
(51) Int. Cl.: C12M 1/00

(54) **Fermenter with preshaped bottom element**
Fermenter mit vorgeformtem Bodenelement
Fermenteur avec élément inférieur préformé

(30) Priority: 26.08.2011 EP 11179094
(43) Date of publication of application: 27.02.2013
(73) Proprietor: Thöni Industriebetriebe GmbH, 6410 Telfs (AT)
(72) Inventor: Schennach, Oliver, 6414 Mieming (AT); Köll, Thomas, 6410 Telfs (AT); Krismer, Michael, 6500 Landeck (AT)
(74) Representative: Dilg, Haeusler, Schindelmann Patentanwaltsgesellschaft mbH

(56) References cited:
- WO-A2-2011/077358
- DE-U1- 20 211 104
- DE-U1-202009 010 166
- GB-A- 1 526 758

## Description

### FIELD OF THE INVENTION

The present invention relates to the field of fermenters for a biogas plant.

### BACKGROUND OF THE INVENTION

IP 0 770 675 B1 discloses a fermenter having a fermentation tank with a steel inner lining. Making a fermentation tank of concrete is difficult due to buoyant forces acting on the formwork.

In view of the above described situation, there exists a need for an improved technique that enables to provide a fermenter while substantially avoiding or at least reducing one or more of the above identified problems.

DE 20 2009 010 166 U1 relates to a fermenter for a biogas plant. The fermenter has fermenter parts attached at a gantry cross beam. In particular at least one floating mixing device and/or at least one associated service platform with a service opening and/or at least one inspection glass are attached to the gantry cross beam.

### SUMMARY OF THE INVENTION

In view of the above described situation, there exists a need for an improved technique that enables to provide a fermenter while substantially avoiding or at least reducing one or more of the above identified problems. This need may be met by the subject matter according to the independent claims. Advantageous embodiments of the herein disclosed subject matter are described by the dependent claims.

The present invention relates to a fermenter according to claim 1 and a method according to claim 14.

According to a first embodiment there is provided a fermenter comprising a wall; and a preshaped bottom element defining the contour of an inner bottom surface of the fermenter; the preshaped bottom element having an outer surface portion, the outer surface portion facing the wall and being laterally spaced from a lower part of the wall.

The usage of a preshaped bottom element for the construction of a fermenter may have the advantage that a wall of the fermenter can be realized and fabricated with a simple formwork while still providing a defined contour of an inner bottom surface of the fermenter.

According to an embodiment, the wall is a vertical straight wall to which the preshaped bottom element is attached. According to an embodiment and depending on the size of the fermenter, the whole bottom of the fermenter may include a single preshaped bottom element or maybe formed of a plurality of preshaped bottom elements, for example two or more preshaped bottom elements. According to an embodiment, the preshaped bottom element, i.e. a single preshaped bottom element defines the whole contour between two spaced apart walls of the fermenter. For example, in an embodiment the contour is adapted to a steering device which is to be mounted in the fermenter for steering the fermentation material contained in the fermenter during operation. According to an embodiment, two or more of the preshaped bottom elements are arranged along an axial direction of the steering device. According to an embodiment, the axial direction is arranged parallel to an access of rotation of the steering device. According to a further embodiment, the fermenter is a plug-flow fermenter or is of the plug flow type, wherein material transport occurs along the axial direction of the fermenter.

According to an embodiment of the first aspect, the fermenter further comprises a coupling element coupling the preshaped bottom element to the wall. The coupling element comprises or consists of a flexible part. The flexible part allows a movement of the preshaped bottom element relative to the wall. According to an embodiment the coupling element is made of sheet metal having a first portion attached to the wall and a second portion attached to the preshaped bottom element. According to a further embodiment, the coupling element comprises at least one bend between the first portion and the second portion. For example, in an embodiment the first portion and the second portion are arranged at an angle with respect to each other. In an embodiment, the first portion being attached to the wall at least partially runs parallel to the wall and at least part of the second portion of the coupling element runs parallel to a mounting surface of the preshaped bottom element to which the coupling element is attached.

According to a first embodiment, the wall is made of concrete. According to an embodiment, the wall further comprises an attachment surface facing the preshaped bottom element, the preshaped bottom element being coupled to the attachment surface. According to an embodiment, the coupling of the preshaped bottom element to the attachment surface is perfomed directly, i.e. by directly attaching the preshaped bottom element to the attachment surface. According to a further embodiment, the coupling of the preshaped bottom element to the attachment surface is performed by a third part, for example by a coupling element as described above. According to an embodiment, the attachment surface is provided by an attachment plate anchored in the wall. For example, the attachment plate may be a metal plate, e.g. a steel plate. In other embodiments, the attachment surface is provided by a portion of the wall.

According to an embodiment, the coupling element is a separate element. According to a further embodiment, the coupling element is formed as a part of the preshaped bottom element or as a part of the attachment plate. According to a further embodiment, the preshaped bottom element has an inner surface portion being opposite the outer surface portion and running parallel to the outer surface portion, wherein the inner surface portion defines the contour of the inner bottom surface of the fermenter. Accordingly, the preshaped bottom element may be generally plate shaped or may comprise a generally plate shaped element. According to an embodiment, the preshaped bottom element further comprises a strengthening element for example a rip, a frame, etc. The strengthening element serves to maintain the shape of the preshaped bottom element.

According to an embodiment, the preshaped bottom element comprises a piece of sheet material forming the inner surface portion and the outer surface portion of the preshaped bottom element.

According to a further embodiment where the coupling element, the attachment plate of the wall, and the attachment surface of the preshaped bottom element are made of metal, the attachment of the coupling element to the attachment plate of the wall on the one hand and to the preshaped bottom element on the other hand is performed by welding.

According to a further embodiment, the fermenter comprises a support and the preshaped bottom element has a bearing face resting on the support. For example, in an embodiment the support is a vertical bar. According to a further embodiment, the support is mounted to a ground plate on which the fermenter is established.

According to an embodiment, the support is located in a recess provided on the ground plate and the recess with the support located therein is grouted with a grouting material. The grouting material may be any suitable material, for example concrete, plastic or resin, just to name some examples.

According to a further embodiment, the fermenter comprises a grouting material between the ground plate and the preshaped bottom element. According to an embodiment, the grouting material is provided between the ground plate and a lower portion of the preshaped bottom element or, in another embodiment. For example, a formwork may be provided around the lower portion of the preshaped bottom element and the grouting material may be poured into the formwork. The grouting material provides a two-dimensional bearing face supporting the preshaped bottom element. Accordingly, the grouting material may add stiffness to the preshaped bottom element, in particular in its central portion where the grouting material is provided. In this way, the grouting material between the preshaped bottom element and the ground plate act to maintain the shape of the preshaped bottom element even under heavy loads, for example in case the fermenter is filled during the fermentation operation.

According to a further embodiment, a spacer element is provided between the preshaped bottom element and the wall. According to an embodiment, the spacer element transfers lateral forces from the preshaped bottom element to the wall. According to a further embodiment, the preshaped bottom element further comprises a supporting surface with the spacer being located between the supporting surface and the wall for shoring up the preshaped bottom element against the wall.

In an embodiment where the wall is manufactured before installing the preshaped bottom element, tolerances of the dimensions of the wall, and in particular tolerances of the distance between two opposing walls between which the preshaped bottom element is installed, can be accomplished by the spacer element. For tightness between the preshaped bottom element and the wall there may be provided a sealing element, e.g. in the form of a coupling element as described above. Hence, in an embodiment, the coupling element provides a sealing function. In other embodiments, the coupling element provides additionally or alternatively a force transfer function between the preshaped bottom element and the wall.

According to an embodiment, the fermenter comprises a free space between the preshaped bottom element and the lower part of the wall. A free space between the preshaped bottom element and the wall can be used for multiple purposes. For example, according to an embodiment the free space includes an inoculate line running through the free space. This has the advantage that the inoculate line is arranged inside the (outer) walls of the fermenter. According to a further embodiment, the free space may form an inspection walkway providing for an easy access of the preshaped bottom element or installations, e.g. heaters, mounted thereto.

According to a further embodiment, the fermenter comprises a heater for heating the preshaped bottom element. According to an embodiment, the heater is heat transfer coupled to the preshaped bottom element. For example, in an embodiment, the heater is attached to the preshaped bottom element. According to an embodiment, the preshaped bottom element is adapted for acting as a heating surface. According to a further embodiment, the heater comprises at least two heater loops.

According to a further embodiment, the heater includes a tube which is heat transfer coupled to the preshaped bottom element. In an embodiment, the tube is meander-shaped. According to a further embodiment, the heater comprises a cavity for a heat transferring medium wherein the cavity is heat transfer coupled to the preshaped bottom element. In an embodiment, the cavity is a bag including the heat transferring medium, for example water. Such a cavity may provide the advantage of a large area of the preshaped bottom element that is heated up by the heat transferring medium. The heat transferring medium may be pumped through the bag or may be warmed up by a heat exchanger located in the heat transferring media. The bag may be formed as a separate bag heat transfer coupled to the preshaped bottom element. In another embodiment, the bag is formed by a bag element and the preshaped bottom element itself. In such a case, the bag element may be attached to the preshaped bottom element with a portion of the bag element being spaced from the preshaped bottom element thereby forming the cavity for the heat transferring medium.

According to an embodiment, the fermenter comprises further wall and a connection rod between the wall and the further wall, wherein the connection rod counteracts an outward movement of the wall and the further wall away from each other. Such an outward movement of the wall and the further wall away from each other may occur during filling of the fermenter where the fermentation material applies a hydrostatic pressure to the walls of the fermenter. According to an embodiment, the connection rod supports maintaining the shape of the preshaped bottom element. By limiting the outward movement of the wall, the connection rod also limits an outward movement on the preshaped bottom element following the wall. In this way, deformation forces on the sealing between the wall and the preshaped bottom element, for example on the coupling element, may be reduced or even eliminated.

According to a further embodiment, the fermenter comprises a platform in an upper corner of the fermenter, the platform extending between the wall and an adjacent wall forming an angle with respect to the wall.

According to an embodiment, the platform in the upper corner of the fermenter reinforces the interconnection between the wall and the adjacent wall. According to an embodiment this may also serve to reduce the forces on a coupling element coupling the pre-shaped preshaped bottom element to the wall.

According to an embodiment, the fermenter comprises a flow barrier for blocking surface flow of the fermentation material in the fermenter. The flow barrier extends into the fermentation material and may be of any suitable shape.

According to an embodiment of a second aspect of the herein disclosed subject matter, a method of building a fermenter is provided, the method comprising providing a preshaped bottom element, defining the contour of an inner bottom surface of the fermenter; providing a wall; and coupling the preshaped bottom element to the wall wherein the preshaped bottom element has an outer surface portion, the outer surface portion facing the wall and being laterally spaced from a lower part of the wall.

In the above there has been described and in the following there will be described exemplary embodiments of the subject matter disclosed herein with reference to a fermenter and a method of building a fermenter. It has to be pointed out that of course any combination of features relating the different aspects of the herein disclosed subject matter is also possible. In particular, some embodiments have been or will be described with reference to apparatus type embodiments whereas other embodiments have been or will be described with reference to method type embodiments.

### BRIEF DESCRIPTION OF THE DRAWINGS

Fig. 1 shows a cross-sectional view of a part of a fermenter according to embodiments of the herein disclosed subject matter.
Fig. 2 shows an elevated view of the fermenter of Fig. 1 when viewed from above, indicated by line II-II of Fig. 1.
Fig. 3 shows a further cross-sectional view of a part of the fermenter of Fig. 1.
Fig. 4 shows in part a cross-sectional view of the preshaped bottom element and the heater according line IV-IV of Fig. 3.
Fig. 5 shows a cross-sectional view of the fermenter of Fig. 1 in accordance with further embodiments of the herein disclosed subject matter.
Fig. 6 shows a top view of the fermenter of Fig. 1 in accordance with embodiments of the herein disclosed subject matter.

### DETAILED DESCRIPTION

The illustration in the drawings is schematic. It is noted that in different figures, similar of identical elements are provided with the same reference signs or with reference signs which are different from the corresponding reverence signs only within the first digit or an appended character.

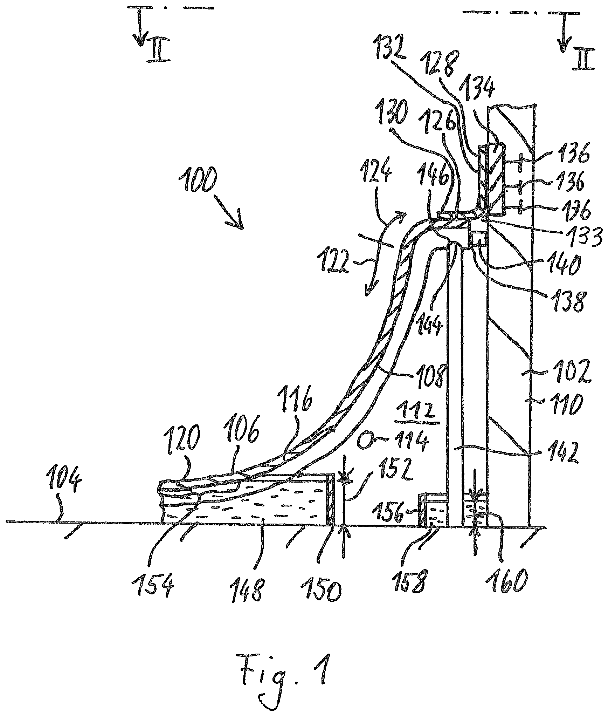

Fig. 1 shows a cross-sectional view of a part of a fermenter according to embodiments of the herein disclosed subject matter.

The fermenter 100 comprises a first wall 102 on a ground plate 104. The fermenter 100 further comprises a preshaped bottom element 106. The preshaped bottom element 106 has an outer surface portion 108 facing the first wall 102 and being laterally spaced from the first wall 102. Although in Fig. 1 the whole outer surface of the preshaped bottom element is laterally spaced from the first wall 102, this is not necessarily the case for any embodiment. Rather, in other embodiments the preshaped bottom element may have a first outer surface portion contacting the wall 102 and a second outer surface portion being laterally spaced from the lower part of the first wall.

Although the wall 102 is referred to as first wall herein, this is not to be understood as limiting but rather for the purpose of identifying the wall 102 from other walls described hereinafter.

In accordance with an embodiment, the outer surface portion 108 which is laterally spaced from the first wall 102 forms a free space between the preshaped bottom element and a lower part 110 of the wall 102. This free space, indicated at 112 in Fig. 1, comprises an inoculate line 114 and is large enough to form an inspection walkway allowing inspection, for example for tightness, maintenance, etc., of the preshaped bottom element 106 or installations attached thereto.

In accordance with an embodiment, the preshaped bottom element comprises plate-shaped part 116, e.g. a piece of sheet metal and a plurality of ribs 118, one of which is shown in Fig. 1. The plate-shaped part 116 defines the contour of an inner bottom surface 120 of the fermenter. According to an embodiment, the contour of the inner bottom surface 120 has a first circular inner portion 122 and a second outer portion 124 which is adapted to be mounted to the wall 102. In Fig. 1 the first portion 122 and the second portion 124 of the inner bottom surface are indicated by respective arrows. In accordance with an embodiment, the first circular portion 102 of the inner bottom surface 120 is adapted to a stirring device mounted in the fermenter for stirring the fermentation material contained in the fermenter. The stirring device is not shown in Fig. 1 for the sake of clarity. According to an embodiment, the stirring device has a rotatable shaft, rotatable about an access of rotation that is arranged perpendicular to the drawing plane of Fig. 1. In such a case the circular inner first portion 122 of the inner bottom surface may be adapted to a circumferential motion path of stirring paddles mounted to the rotatable shaft. According to an embodiment, the second portion 124 of the inner bottom surface 120 has a convex curvature in contrast to the concave curvature of the first circular portion 122. The convex second portion 124 of the contour of the inner bottom surface 120 provides a mounting surface 126 to which a coupling element 128 is attached. However, in other embodiments, the mounting surface 126 may have another orientation or may be provided at another location on the preshaped bottom element as long as it allows attachment of a respectively shaped coupling element 128.

In accordance with an embodiment, the coupling element 126 forms or comprises a flexible part providing some flexibility between the preshaped bottom element 106 and the first wall 102. In accordance with an embodiment, the coupling element 128 comprises a first portion 130 which is attached to the attachment surface 126 of the preshaped bottom element. The coupling element 128 further comprises the second portion 132 which is coupled to the wall 102. The wall 102, in accordance with an embodiment, is made of concrete. In accordance with an embodiment, the first wall 102 further comprises an attachment surface 133 to which the coupling element 128 is attached. According to an embodiment, the attachment surface 133 is provided by an attachment plate 134 which is mounted to the first wall 102, e.g. with anchors 136. According to an embodiment, the anchors 136 are head bolt dowels. However, depending on the type of the first wall 102, any suitable attachment element may be used for mounting the attachement plate 134 to the first wall 102. In accordance with an embodiment, the attachment plate 134 is a metal plate, e.g. a steel plate. The use of metal as a material for the attachment surface 133, for the coupling element 128 and for the mounting surface 126 of the preshaped bottom element allows an attachment of the coupling element 128 to the attachment surface 133 of the wall and to the mounting surface of the preshaped bottom 106 element by welding.

In accordance with an embodiment, the preshaped bottom element 106 comprises a lateral supporting surface 138, the lateral supporting surface facing the first wall 102. Further the fermenter 100 comprises a spacer 140, the spacer being located between the supporting surface 138 and the first wall 102. In accordance with an embodiment, the spacer 140 is adapted for shoring up the preshaped bottom element 106 against the wall 102. In other words, the spacer 140 transfers lateral forces acting on the preshaped bottom element to the wall 102 and vice versa.

The spacer 140 is in particular suitable for an easy assembly of the fermenter 100 as it provides for an easy compensation of manufacturing tolerances of the preshaped bottom element 106 and/or the wall 102.

According to an embodiment, the fermenter 100 comprises a support 142. The preshaped bottom element 106 as a bearing face 144 resting on the support 142. In accordance with an embodiment, the support 142 extends from the bearing face 144 to the ground plate 104. According to other embodiments, the support 142 is attached to a further suitable component of the fermenter, for example to the wall 102.

According to an embodiment, the bearing face 144 has a curved shape and the support has respectively formed opposing shape such that the preshaped bottom element is pivotable with respect to the support. Such a configuration, wherein the bearing face 144 and the opposing face on the support provide for the preshaped bottom element 106 being pivotable with respect to the support 142 can reduce shearing forces on the support 142. According to an embodiment, the bearing face 144 is a spherical face.

According to a further embodiment, the bearing face 144 is a flat face and the opposing face 146 of the support 142 is also a flat face slidingly engaging the bearing face 144 of the preshaped bottom element 106. According to still other embodiments, the support 142 may be attached to the preshaped bottom element 106, e.g. by welding.

In accordance with the further embodiment, the fermenter 100 comprises a grouting material 148 between the ground plate 104 and the preshaped bottom element 106. For example, according to an embodiment a formwork 150 is provided in a center portion of the fermenter 100 and the grouting material is poured into the formwork 150, thereby filling the space between the ground plate 104 and the preshaped bottom element 106 within the formwork 150 up to a predetermined level 152 above the ground plate 104. According to an embodiment, the grouting material is concrete. However, according to other embodiments other materials are also suitable as grouting material if these materials are capable of bearing the temperature generated during fermentation and the load exerted by the preshaped bottom element if the fermenter is filled with fermentation material (not shown in Fig. 1). The grouting material 148 has the advantage of providing a large surface 154 of the preshaped bottom element 106 to be supported by the grouting element 148. Further, the grouting material may serve to maintain the shape of the preshaped bottom element under load, e.g. if the fermenter is filled with fermentation material exerting a hydrostatic pressure on the preshaped bottom element.

The support 142 may also be grouted in a respective formwork 156 by means of a grouting material 158. Again the grouting material 158 fills the formwork 156 up to a predetermined level 160 above the ground plate 104.

It should be understood that any formwork mentioned herein, e.g. the formwork 150 or the formwork 156 may be provided by a construction monted on a surface, e.g. on a surface of the ground plate 104, or, in other embodiments, may be provided by a recess formed in a surface, e.g. in the surface of the ground plate 104.

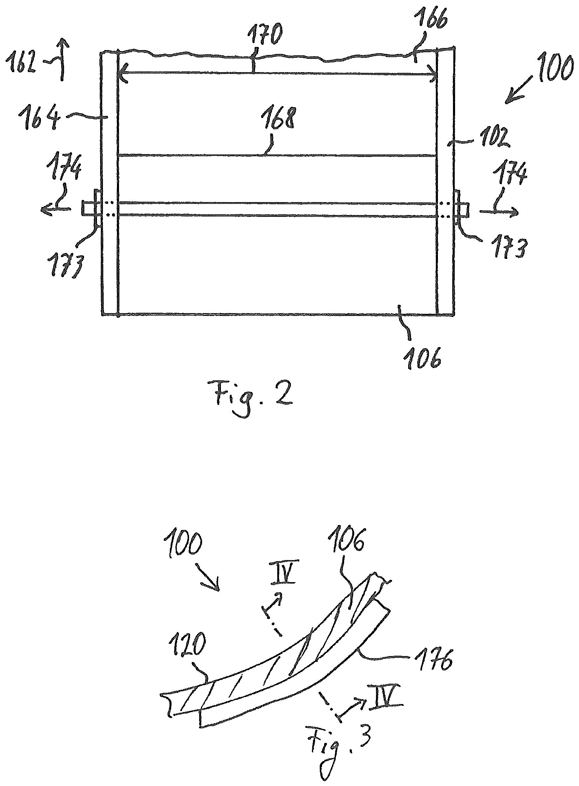

Fig. 2 shows an elevated view of the fermenter 100 when viewed from above, indicated by line II-II of Fig. 1.

It is noted that in Fig. 2 the fermenter 100 is not fully shown in its axial direction indicated as 162 in Fig. 2. In Fig. 2 the first wall 102 and an opposing second wall 164 of the fermenter is shown. Between the opposing walls 102, 164 the preshaped bottom element 106 is arranged. Further shown in Fig. 2 is a further preshaped bottom element 166. In the preshaped bottom elements 106, 166 are attached to each other, for example by welding at a weld line 168. In accordance with an embodiment, each preshaped bottom element 106, 166 is configured for extending the whole width 170 between the two opposing walls 102, 164. This makes it possible to manufacture the preshaped bottom element with the full contour of the inner bottom surface of the fermenter remote from the installation site thereby facilitating at the assembly of the fermenter 100 at the installation site. A first embodiment discloses a method of building a fermenter according to claim 14. The method further comprises providing the wall 102, and an opposing wall 164. Thereafter, the preshaped bottom element 106 between the opposing walls 102, 164 is provided. Subsequently, the further preshaped bottom element 166 is provided between the opposing walls 102, 164. It is noted that providing a further preshaped bottom element is repeated if necessary, depending on the length of the fermenter 100 in axial direction 162. The method of building the fermenter 100 further comprises the step of attaching to adjacent preshaped bottom elements 106, 166 to each other. Further, according to an embodiment the method further comprises providing the spacers 140 (is not shown in Fig. 2) between the supporting surface 138 of the preshaped bottom element and its opposing wall 102. It is noted, that according to an embodiment the width of the spacer 140 is selected so as to bridge the space between the supporting surface 138 and the wall 102. Due to tolerances occurring during manufacture of the components of the fermenter, in particular of the preshaped bottom element 106 and/or the wall 102, the width of the spacer 140 is in an embodiment adapted to bridge the actual distance between the supporting surface 138 and the wall 102. In accordance with an embodiment, the supporting surface 138 is provided by a rib 118 attached to a plate-shaped part 116 of the preshaped bottom element 106. The method comprises attaching a coupling element 128 to the preshaped bottom element and to the wall 102, thereby sealingly coupling the preshaped bottom element 106 and the attachment surface 133 of the wall 102. According to a further embodiment, the method of building a fermenter comprises providing a grouting material 148 between the preshaped bottom element 106 and the ground plate 104. According to a further embodiment, the method of building a fermenter comprises providing the support 142 for supporting the bearing face 144 of the preshaped bottom element 106. According to a further embodiment, the method comprises grouting the support 144 in a grouting material 158 thereby fixing the spatial position of the support 142.

Now referring back to Fig. 2, according to an embodiment the fermenter 100 comprises a connection rod 172 between the opposing walls 102, 164. According to an embodiment, the connection rod 172 counteracts an outwardly directed force acting on the wall 102 and the further wall 164 in a direction away from each other. Such an outwardly directed force indicated at 174 in Fig. 2, may occur due to the hydrostatic pressure of fermentation material filling the fermenter 100.

According to an embodiment, the connection rod 172 extends through the walls 102, 164 and is secured to the respective wall by suitable means, e.g. by a threaded nut 173. According to other embodiments, the connection rod extends between respective attachment plates on the wall 102, 164, the attachment plates facing each other, i.e. the attachment plates are provided on an inner surface of the wall 102, 164.

Fig. 3 shows in part a further cross-sectional view of the fermenter 100 of Fig. 1.

According to an embodiment, a heater 176 for heating the preshaped bottom element 106 is provided. According to an embodiment, the heater 176 is attached to the preshaped bottom element. According to further embodiments, the heater 176 may be configured for contactless heating of the preshaped bottom element 106, e.g. by induction. According to further embodiments, the heater 176 comprises a tube with a heating medium flowing there through. According to an embodiment, the tube may be meander-shaped. According to a further embodiment, a cavity may be provided, the cavity containing a heat transfer medium which is heated by circulating the heat transfer medium through the cavity or by heating the heat transfer medium by suitable means, e.g. a heat exchanger. According to an embodiment, the heat exchanger may comprise one or more tubes through which a heat transfer medium is circulated.

In an embodiment, where the preshaped bottom element is made of metal, e.g. made of steel, the preshaped bottom element itself provides for a suitable heat transfer characteristics from the heater 176 to the inner bottom surface 120 of the preshaped bottom element. According to an embodiment, the cavity containing the heat transfer medium is made of a separate part fully encapsulating the heat transfer medium and being attachable or attached to the preshaped bottom element 106. According to another embodiment, the preshaped bottom element 106 forms a wall portion of the cavity which contains the heat transfer medium.

In an embodiment, where a free space or an inspection walkway is formed between the preshaped bottom element 106 and the wall 102, monitoring of the heater and/or maintenance and/or repair of the heater is easily performed.

If the preshaped bottom element 106 is made of metal, a good heat conduction between the heater 176 and the inner bottom surface 120 (and hence to the content of the fermenter) is provided.

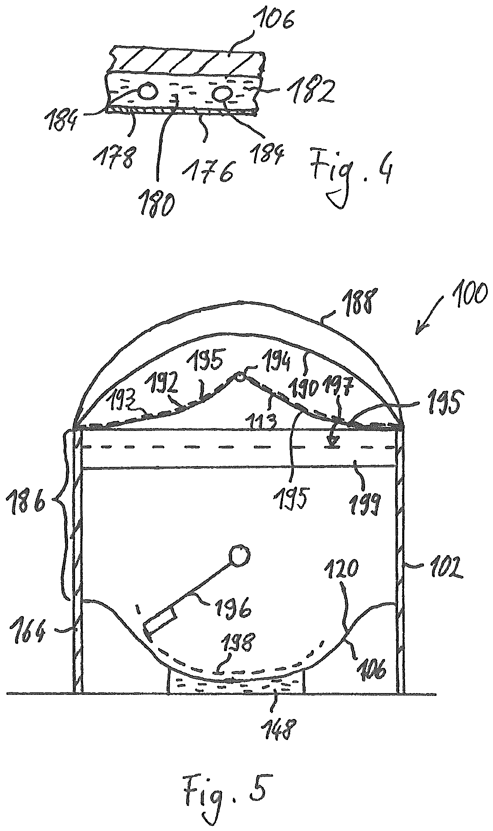

Fig. 4 shows in part a cross-sectional view of the preshaped bottom element and the heater 176 according line IV-IV of Fig. 3.

Fig. 4 shows the preshaped bottom element 106 and a concave bag portion 178 sealingly attached to the preshaped bottom element 106, thereby forming a cavity 180 which is filled with the heat transfer medium 182. Further shown in Fig. 4 are two tube portions 184 through which a temper fluid is circulated, thereby tempering the heat transfer medium 182 and hence, the preshaped bottom element 106.

It should be noted that instead of the configuration of the heater 176 shown in Fig. 4 any other suitable heater configuration is also possible, e.g. in accordance with an embodiment described herein. Moreover, a person skilled in the art is aware of the fact that many other heater configurations may be used in conjunction with the preshaped bottom element according to embodiments of the herein disclosed subject matter.

Fig. 5 shows a cross-sectional view of the fermenter 100 of Fig. 1 in accordance with further embodiments of the herein disclosed subject matter.

Fig. 5 again shows the walls 102, 164 and the preshaped bottom element 106 extending therebetween, the preshaped bottom element being supported in its central portion by a grouting material 148. In accordance with an embodiment, the walls from 102, 164 are solid walls having an upper part 186 extending above in the preshaped bottom element and laterally defining the fermenter volume. In accordance with an embodiment, the roof 188 of the fermenter 100 is an air supported roof. Further shown in Fig. 1 is a gas membrane extending between the walls 102, 164 below the roof 188. In Fig. 5 the gas membrane is shown in an inflated state corresponding to a certain amount of biogas being contained below the gas membrane 190. If there is less gas above the fermentation material indicated at 192 in Fig. 5, the gas membrane 190 rests on a membrane support 193 which prevents the gas membrane 190 from being contacted by the stirring device exemplarily indicated at 196 in Fig. 5. According to an embodiment the membrane support 193 is formed by a center bar 194 and belts 195 extending from the center bar 194 to the walls 102, 164.

As mentioned above and as can be seen in Fig. 5, the contour of the inner bottom surface 120 defined by the preshaped bottom element 106 is adapted to a circumferential motion path 198 of the stirring device 196.

In accordance with an embodiment, the fermenter 100 is a plug flow fermenter. However, aspects and embodiments of the herein disclosed subject matter are at least partly also applicable to other forms of fermenters.

According to an embodiment, the fermenter comprises a flow barrier 199 for blocking or at least reducing a surface flow of the fermentation material in the fermenter. The flow barrier 199 extends into the fermentation material, e.g. downwards below the rated filling level 197 of the fermenter. According to an embodiment, the flow barrier extends between two opposing walls 102, 164 transverse to the prinzipal flow direction of the fermentation material, e.g. transverse to the axial direction in case of a plug flow fermenter. The blocking of surface flow of the fermentation material may increase the residence time of the fermentation material in the fermenter as it prevents or reduces a short circuit surface flow.

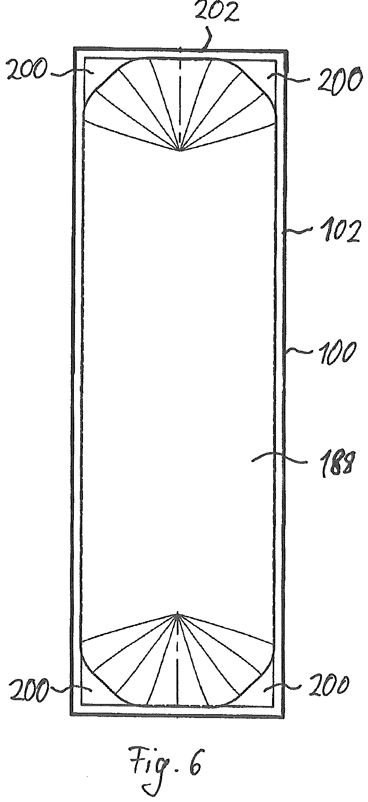

Fig. 6 shows a top view of the fermenter 100 in accordance with embodiments of the herein disclosed subject matter.

According to an embodiment, the fermenter 100 comprises a platform 200 in at least one upper corner of the fermenter 100, the platform extending in between a first wall 102 and an adjacent wall 202 formed at an angle with respect to the first wall 102. Fig. 6 shows four such platforms 200 in the four corners of the fermenter 100. The platforms 200 may be configured for strengthening the corner joints of the fermenter walls. According to other embodiments, the platforms 200 allow an easy installation of measurement apparatuses and further provide access to the fermenter from above. In other embodiments, following the principles of disclosure of the herein disclosed subject matter, the platforms may be configured for providing both functions, strengthening the corner joints and allow an easy installation of measurement apparatuses.

It should be noted that any entity or element as disclosed herein is not limited to the dedicated entities described in some embodiments. Rather, the herein disclosed subject matter may be implemented in various ways, in various locations and at various granularities with respect to the entities and elements described, while still providing the desired functionality. For example, a wall described herein may be provided as single piece or may be provided as two or more pieces joined together. Further, the preshaped bottom element may be for example formed of by a single piece of sheet metal or may be formed by two or more pieces of sheet metal which are joined together, preformed, reinforced by strengthening elements, such as ribs, etc.

It should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

In order to recapitulate the above described embodiments of the present invention one can state:

It is described a fermenter, comprising a wall; and a preshaped bottom element defining the contour of an inner bottom surface of the fermenter. The preshaped bottom element has an outer surface portion, the outer surface portion facing the wall and being laterally spaced from a lower part of the wall. The fermenter may be built by providing lateral walls, placing the preshaped bottom element between the lateral walls and providing a sealing element between the preshaped bottom element and each of the walls.

Embodiments of the herein disclosed subject matter allow for small tolerances for the contour of the inner bottom surface of the fermenter and only low deviations from the predefined contour under the load of the fermentation material. Therefore the gap between the contour of the inner bottom surface of the fermenter and the stirring apparatus can be small, resulting in small amounts of sediment forming at the bottom of the fermenter.

### List of reference signs

- 100: fermenter
- 102: first wall
- 104: ground plate
- 106: preshaped bottom element
- 108: outer surface portion
- 110: lower part of 102
- 112: free space
- 114: inoculate line
- 116: plate-shaped part
- 118: rib
- 120: inner bottom surface
- 122: first inner portion of 106
- 124: second outer portion of 106
- 126: mounting surface
- 128: coupling element
- 130: first portion of 128
- 132: second portion of 128
- 133: attachment surface
- 134: attachment plate
- 136: anchor
- 138: lateral supporting surface
- 140: spacer
- 142: support
- 144: bearing face of 106
- 146: opposing face 146 of 142, opposing 144
- 148: grouting material
- 150: formwork
- 152: predetermined level
- 154: surface of 106 which is supported by 148
- 156: formwork
- 158: grouting material
- 160: predetermined level
- 162: axial direction
- 164: second wall
- 166: further preshaped bottom element
- 168: weld line
- 170: width between two opposing walls
- 172: connection rod
- 173: threaded nut
- 174: outwardly directed force
- 176: heater
- 178: bag portion
- 180: cavity
- 182: heat transfer medium
- 184: tube portion
- 186: upper part of 102, 164
- 188: roof
- 190: gas membrane
- 192: gas membrane with less biogas below the gas membrane as in the state at 190
- 193: membrane support
- 194: center bar
- 195: belt
- 196: stirring device
- 197: rated filling level of the fermenter
- 198: motion path of 196
- 199: flow barrier
- 200: platform between ajacent walls
- 202: wall adjacent to 102

## Claims

1. Fermenter (100), comprising:
- a wall (102, 164); and
- a preshaped bottom element (106) defining the contour of an inner bottom surface (120) of the fermenter (100);
- the preshaped bottom element (106) having an outer surface portion (108), the outer surface portion (108) facing the wall (102, 164) and being laterally spaced from a lower part of the wall (102, 164);
- the preshaped bottom element (106) having an inner surface portion (120) being opposite the outer surface portion (108) and running parallel to the outer surface portion (108);
- the inner surface portion (120) defining the contour of the inner bottom surface of the fermenter (100);
- the inner surface portion (120) and the outer surface portion (108) being formed by a piece of sheet metal; and
- the wall (102, 164) being made of concrete;
- the fermenter further comprising a coupling element (128) coupling the preshaped bottom element (106) to the wall (102, 164);
- the coupling element (128) allowing a movement of the preshaped bottom element (106) relative to the wall (102, 164).

2. Fermenter (100) according to claim 1,
the coupling element being made of sheet metal having a first portion attached to the wall and a second portion attached to the preshaped bottom element.

3. Fermenter according to any one of claims 1 or 2,
the coupling element being a separate element.

4. Fermenter according to any one of claims 1 or 2,
the coupling element being formed as a part of the preshaped bottom element or as a part of an attachment plate.

5. Fermenter (100) according to any one of claims 1 to 4, further comprising
- a support (142);
- the preshaped bottom element (106) having a bearing face (144) resting on the support (142).

6. Fermenter (100) according to any one of claims 1 to 5, the wall (102, 164) further comprising an attachment surface (133) facing the preshaped bottom element (106), the preshaped bottom element (106) being coupled to the attachment surface (133).

7. Fermenter (100) according to any one of the preceding claims, further comprising:
- a ground plate (104); and
- a grouting material (148) between the ground plate (104) and the preshaped bottom element (106).

8. Fermenter (100) according to any one of the preceding claims, further comprising a free space (112) between the preshaped bottom element (106) and the lower part of the wall (102, 164).

9. Fermenter (100) according to claim 8, further comprising an inoculate line (114) in the free space (112).

10. Fermenter (100) according to one of the preceding claims,
- the preshaped bottom element (106) further comprising a supporting surface (138);
- the fermenter (100) comprising a spacer (140) located between the supporting surface (138) and the wall (102, 164) for shoring up the preshaped bottom element (106) against the wall (102, 164).

11. Fermenter (100) according to any one of the preceding claims, further comprising a heater (170) for heating the preshaped bottom element (106).

12. Fermenter (100) according to any one of the preceding claims, further comprising:
- a further wall (164);
- a connection rod (172) between the wall (102) and the further wall (164); the connection rod (172) counteracting an outwardly directed force acting on the wall (102) and the further wall (164) in a direction away from each other.

13. Fermenter (100) according to any one of the preceding claims, further comprising a platform (200) in an upper corner of the fermenter (100), the platform (200) extending between the wall (102, 164) and an adjacent wall (202) formed at an angle with respect to the wall (102, 164).

14. Method of building a fermenter (100), the method comprising:
- providing a preshaped bottom element (106) defining the contour of an inner bottom surface (120) of the fermenter (100), the preshaped bottom element (106) having an outer surface portion (108), the preshaped bottom element (106) having an inner surface portion (120) being opposite the outer surface portion (108) and running parallel to the outer surface portion (108), the inner surface portion (120) defining the contour of the inner bottom surface of the fermenter (100), and the inner surface portion (120) and the outer surface portion (108) being formed by a piece of sheet metal;
- providing a wall (102, 164) made of concrete;
- coupling the preshaped bottom element (106) to the wall (102, 164) by a coupling element; the outer surface portion (108) facing the wall (102, 164) and being laterally spaced from a lower part of the wall (102, 164); and the coupling element (128) allowing a movement of the preshaped bottom element (106) relative to the wall (102, 164).

## Patentansprüche

1. Fermenter (100) aufweisend:
- eine Wand (102, 164); und
- ein vorgeformtes Bodenelement (106), welches die Kontur von einer inneren Bodenoberfläche (120) von dem Fermenter (100) definiert;
- wobei das vorgeformte Bodenelement (106) ein äußeres Oberflächenteil (108) hat, wobei das äußere Oberflächenteil (108) der Wand (102, 164) zugewandt ist und lateral von einem unteren Teil von der Wand (102, 164) beabstandet ist;
- wobei das vorgeformte Bodenelement (106) ein inneres Oberflächenteil (120) hat, welches dem äußeren Oberflächenteil (108) gegenüberliegt und parallel zu dem äußeren Oberflächenteil (108) verläuft;
- wobei das innere Oberflächenteil (120) die Kontur von der inneren Bodenoberfläche von dem Fermenter (100) definiert;
- wobei der innere Oberflächenteil (120) und der äußere Oberflächenteil (108) mittels eines Stückes aus Metallblech geformt sind; und
- wobei die Wand (102, 164) aus Beton besteht;
- wobei der Fermenter ferner aufweist ein Kopplungselement (128), welches das vorgeformte Bodenelement (106) mit der Wand (102, 164) koppelt;
- wobei das Kopplungselement (128) eine Bewegung von dem vorgeformten Bodenelement (106) relativ zu der Wand (102, 164) erlaubt.

2. Fermenter (100) gemäß Anspruch 1,
wobei das Kopplungselement aus Metallblech besteht, welches einen ersten Teil, befestigt an der Wand, und einen zweiten Teil, befestigt an dem vorgeformten Bodenelement, hat.

3. Fermenter gemäß einem der Ansprüche 1 oder 2,
wobei das Kopplungselement ein separates Element ist.

4. Fermenter gemäß einem der Ansprüche 1 oder 2,
wobei das Kopplungselement als ein Teil von dem vorgeformten Bodenelement oder als ein Teil von einer Apparateplatte geformt ist.

5. Fermenter (100) gemäß einem der Ansprüche 1 bis 4, ferner aufweisend
- eine Stütze (142);
- wobei das vorgeformte Bodenelement (106) eine Auflagefläche (144) hat, welche auf der Stütze (142) aufliegt.

6. Fermenter (100) gemäß einem der Ansprüche 1 bis 5, wobei die Wand (102, 164) ferner aufweist eine Befestigungsoberfläche (133), welche dem vorgeformten Bodenelement (106) zugewandt ist, wobei das vorgeformte Bodenelement (106) mit der Befestigungsoberfläche (133) gekoppelt ist.

7. Fermenter (100) gemäß einem der vorhergehenden Ansprüche, ferner aufweisend:
- eine Bodenplatte (104); und
- ein Vergussmaterial (148) zwischen der Bodenplatte (104) und dem vorgeformten Bodenelement (106).

8. Fermenter (100) gemäß einem der vorhergehenden Ansprüche, ferner aufweisend einen freien Raum (112) zwischen dem vorgeformten Bodenelement (106) und dem unteren Teil von der Wand (102, 164).

9. Fermenter (100) gemäß Anspruch 8, ferner aufweisend eine inokulierte Linie (114) in dem freien Raum (112).

10. Fermenter (100) gemäß einem der vorhergehenden Ansprüche,
- wobei das vorgeformte Bodenelement (106) ferner eine Stützoberfläche (138) aufweist;
- wobei der Fermenter (100) einen Abstandshalter (140) aufweist, welcher zwischen der Stützoberfläche (138) und der Wand (102, 164) angeordnet ist zum Abstützten des vorgeformten Bodenelementes (106) gegen die Wand (102, 164).

11. Fermenter (100) gemäß einem der vorhergehenden Ansprüche, ferner aufweisend einen Heizer (170) zum Erhitzen des vorgeformten Bodenelementes des (106).

12. Fermenter (100) gemäß einem der vorhergehenden Ansprüche, ferner aufweisend:
- eine weitere Wand (164);
- eine Verbindungsstange (172) zwischen der Wand (102) und der weiteren Wand (164); wobei die Verbindungstange (172) einer nach außen gerichteten Kraft entgegenwirkt, welche auf die Wand (102) und die weitere Wand (164) in einer Richtung entfernt voneinander wirkt.

13. Fermenter (100) gemäß einem der vorhergehenden Ansprüche, ferner aufweisend eine Plattform (200) in einer oberen Ecke von dem Fermenter (100), wobei die Plattform (200) sich zwischen der Wand (102, 164) und einer angrenzenden Wand (202), welche in einem Winkel in Bezug auf die Wand (102 , 164) geformt ist, erstreckt.

14. Verfahren zum Bauen eines Fermenters (100), wobei das Verfahren aufweist:
- Bereitstellen eines vorgeformten Bodenelementes (106), welches die Kontur von einer inneren Bodenoberfläche (120) von dem Fermenter (100) definiert, wobei das vorgeformte Bodenelement (106) ein äußeres Oberflächenteil (108) hat, wobei das vorgeformte Bodenelement (106) ein inneres Oberflächenteil (120) hat, welches dem äußeren Oberflächenteil (108) gegenüberliegt und parallel zu dem äußeren Oberflächenteil (108) verläuft; wobei das innere Oberflächenteil (120) die Kontur von der inneren Bodenoberfläche von dem Fermenter (100) definiert; und der innere Oberflächenteil (120) und der äußere Oberflächenteil (108) mittels eines Stückes von Metallblech geformt sind;
- Bereitstellen einer Wand (102, 164), welche aus Beton besteht;
- Koppeln des vorgeformten Bodenelementes (106) mit der Wand (102, 164) mittels eines Kopplungselementes; wobei das äußere Oberflächenteil (108) der Wand (102, 164) zugewandt ist und lateral von einem unteren Teil von der Wand (102, 164) beabstandet ist und das Kopplungselement (128) eine Bewegung von dem vorgeformten Bodenelement (106) relativ zu der Wand (102, 164) erlaubt.

## Revendications

1. Fermenteur (100), comprenant :
- une paroi (102, 164) ; et
- un élément inférieur préformé (106) définissant le contour d'une surface inférieure intérieure (120) du fermenteur (100) ;
- l'élément inférieur préformé (106) ayant une portion de surface extérieure (108), la portion de surface extérieure (108) faisant face à la paroi (102, 164) et étant latéralement espacée d'une partie inférieure de la paroi (102, 164) ;
- l'élément inférieur préformé (106) ayant une portion de surface intérieure (120) opposée à la portion de surface extérieure (108) et s'étendant parallèlement à la portion de surface extérieure (108) ;
- la portion de surface intérieure (120) définissant le contour de la surface inférieure intérieure du fermenteur (100) ;
- la portion de surface intérieure (120) et la portion de surface extérieure (108) étant formées d'une pièce de tôle ; et
- la paroi (102, 164) étant réalisée en béton ;
- le fermenteur comprenant en outre un élément de couplage (128) couplant l'élément inférieur préformé (106) à la paroi (102, 164) ;
- l'élément de couplage (128) permettant un mouvement de l'élément inférieur préformé (106) par rapport à la paroi (102, 164).

2. Fermenteur (100) selon la revendication 1,
l'élément de couplage étant réalisé en tôle ayant une première portion fixée à la paroi et une deuxième portion fixée à l'élément inférieur préformé.

3. Fermenteur selon l'une quelconque des revendications 1 ou 2,
l'élément de couplage étant un élément séparé.

4. Fermenteur selon l'une quelconque des revendications 1 ou 2,
l'élément de couplage étant formé comme une partie de l'élément inférieur préformé ou comme une partie d'une plaque de fixation.

5. Fermenteur (100) selon l'une quelconque des revendications 1 à 4, comprenant en outre
- un support (142) ;
- l'élément inférieur préformé (106) ayant une face d'appui (144) reposant sur le support (142).

6. Fermenteur (100) selon l'une quelconque des revendications 1 à 5, la paroi (102, 164) comprenant en outre une surface de fixation (133) faisant face à l'élément inférieur préformé (106), l'élément inférieur préformé (106) étant couplé à la surface de fixation (133).

7. Fermenteur (100) selon l'une quelconque des revendications précédentes, comprenant en outre :
- une plaque de base (104) ; et
- un matériau de jointoiement (148) entre la plaque de base (104) et l'élément inférieur préformé (106).

8. Fermenteur (100) selon l'une quelconque des revendications précédentes, comprenant en outre un espace libre (112) entre l'élément inférieur préformé (106) et la partie inférieure de la paroi (102, 164).

9. Fermenteur (100) selon la revendication 8, comprenant en outre une ligne inoculée (114) dans l'espace libre (112).

10. Fermenteur (100) selon l'une quelconque des revendications précédentes,
- l'élément inférieur préformé (106) comprenant en outre une surface de support (138) ;
- le fermenteur (100) comprenant une entretoise (140) située entre la surface de support (138) et la paroi (102, 164) pour étayer l'élément inférieur préformé (106) contre la paroi (102, 164).

11. Fermenteur (100) selon l'une quelconque des revendications précédentes, comprenant en outre un dispositif de chauffage (170) pour chauffer l'élément inférieur préformé (106).

12. Fermenteur (100) selon l'une quelconque des revendications précédentes, comprenant en outre :
- une paroi supplémentaire (164) ;
- une tige de connexion (172) entre la paroi (102) et la paroi supplémentaire (164) ; la tige de connexion (172) contrecarrant une force dirigée vers l'extérieur agissant sur la paroi (102) et la paroi supplémentaire (164) dans une direction d'éloignement l'une de l'autre.

13. Fermenteur (100) selon l'une quelconque des revendications précédentes, comprenant en outre une plate-forme (200) dans un coin supérieur du fermenteur (100), la plateforme (200) s'étendant entre la paroi (102, 164) et une paroi adjacente (202) formée à un angle par rapport à la paroi (102, 164).

14. Procédé de construction d'un fermenteur (100), le procédé comprenant :
- la fourniture d'un élément inférieur préformé (106) définissant le contour d'une surface inférieure intérieure (120) du fermenteur (100), l'élément inférieur préformé (106) ayant une portion de surface extérieure (108), l'élément inférieur préformé (106) ayant une portion de surface intérieure (120) opposée à la portion de surface extérieure (108) et s'étendant parallèlement à la portion de surface extérieure (108), la portion de surface intérieure (120) définissant le contour de la surface inférieure intérieure du fermenteur (100), et la portion de surface intérieure (120) et la portion de surface extérieure (108) étant formées d'une pièce de tôle ;
- la fourniture d'une paroi (102, 164) réalisée en béton ;
- le couplage de l'élément inférieur préformé (106) à la paroi (102, 164) par un élément de couplage ; la portion de surface extérieure (108) faisant face à la paroi (102, 164) et étant latéralement espacée d'une partie inférieure de la paroi (102, 164) ; et l'élément de couplage (128) permettant un mouvement de l'élément inférieur préformé (106) par rapport à la paroi (102, 164).
